# EUROPEAN PATENT SPECIFICATION

(11) **EP 1 914 015 B1**
(45) Date of publication and mention of the grant of the patent: **24.08.2016**
(21) Application number: 06425718.1
(22) Date of filing: 19.10.2006
(51) Int. Cl.: B09C 1/00, G01N 33/24

(54) **Method based on the use of a gas mixture for sizing systems of gas diffusion in groundwater and evaluating the aquifer contamination**
Verfahren basierend auf der Verwendung einer Gasmischung zur Dimensionierung von Systemen für die Gasverteilung in Grundwasser und Auswertung der Grundwasserleiterverschmutzung
Procédé basé sur l'utilisation d'un mélange gazeux pour dimensionner des systèmes de diffusion de gaz dans les eaux souterraines et évaluer la contamination de l'aquifère

(43) Date of publication of application: 23.04.2008
(73) Proprietor: SOCIETA' ITALIANA ACETILENE E DERIVATI S.I.A.D. S.p.A. in abbreviated form SIAD S.p.A., 24126 Bergamo (IT)
(72) Inventor: Bissolotti, Giorgio, 24126 Bergamo (IT); Pasinetti, Eleonora, 24126 Bergamo (IT); Peroni, Michela, 24126 Bergamo (IT)
(74) Representative: Minoja, Fabrizio

(56) References cited:
- WO-A-2006/004567
- NL-C2- 1 007 420
- US-A- 5 992 213
- US-A- 6 003 365
- US-A1- 2002 067 953

## Description

### Background of the invention

Legislation about ground and groundwater reclamation has been paying particular attention to *in situ* technologies, because these technologies do not require disposal or extraction of soil and/or water.

In Italy, unfortunately, most of remediation systems still remain bound to extraction/exportation and disposal. These systems are very expensive and produce huge quantities of waste that need to be transported and disposed of. In particular, in many cases, the disposal system used involves stocking the polluted ground in authorised rubbish dump.

These reclamation systems result to have a great environmental impact.

One of the technologies widely used to treat groundwater is *Pump & Treat,* that consists of pumping and treating the groundwater on site. It requires great economic resources and it can last many years before giving significant contamination reduction. The system often does not reach the pollutant limits target because the undissolved pollution substances present in the soil are slowly released and solubilised in groundwater, thus determining long pollution tails hard to remove unless other technologies, involving however higher costs, are used.

The treatment of pumped water is usually made on site, and needs the installation of big decontamination plants that often do not allow to leave the site active.

The treated water, moreover, has to be sent to disposal as waste because the discharge on superficial water is often not allowed, involving additional costs.

For these reasons, the application of *in situ* technologies, widely spread in the USA, begins to be of interest in Italy too, because of its undoubted economic and efficiency advantages.

Among these technologies the most widespread is *Air-Sparging.* This remediation system consists in injecting air in the soil using compressors with flow of 40-60 m³/h, in order to remove the contamination of the saturated and unsaturated ground fraction through biologic degradation and stripping processes.

*Air Sparging* is usually combined with a vapour extraction process to recover stripped volatile substances and subsequently subject them to treatment. The remediation system requires, therefore, the provision of a large number of (injection and suction) wells, the use of compressors and a vapours treatment system installation.

As such, besides having to be electrically powered, *Air-Sparging* involves significant on site physical obstacles and the application costs are often not competitive.

Efficiency of Air Sparging depends strictly on ground characteristics and it is very important to pay attention to preferential way that could avoid the homogeneous recovering of vapours.

Air Sparging is applicable when the ground depth is not greater than 15-20 m from the piezometric level, since deeper applications require powerful compression systems with high energy consumption, that makes the treatment economically not competitive.

In order to reduce stripping processes and improve biological processes, decontamination of saturated ground fraction could involve the reduction of air injection till 8-15 m³/h: in this case the technology is called *biosparging.*

In case of volatile substances contamination, as light hydrocarbons, *biosparging* could involve stripping phenomena too, since gas flows remain significant, diffusion systems are not very efficient and the use of air does not optimise the oxygen dissolution in water. For these reasons *biosparging* often requires combined vapour extraction and treatment systems, with consequent increase of economic and managerial costs increase. Moreover, *Biosparging* requires the use of compressors too, so it needs electric power and a place where to install the machines. The use of air allows to reach oxygen concentration in groundwater of 8-9 mgO₂/l at most and so the oxygen diffusion is slow and difficult.

In the last years, especially in the USA, new injection systems using pure oxygen instead of air have been introduced. Pure oxygen allows to reach concentrations higher than 40-60 mgO₂/l depending on the depth of the water column and on temperature. Gas diffusion phenomena can be increased thanks to high concentration gradients, moreover gas flows can be reduced with respect to the air, thus decreasing stripping processes of volatile substances present in groundwater.

The diffusion equipment has a great importance on the gas dissolution efficiency. In particular, the more gas-liquid exchange surface increases, the more efficient the oxygen dissolution in groundwater. The smaller the gas bubbles, the better the gas dissolution.

One of the existing systems that apply this principle is ISOC™ (*In Situ Submerged Oxygen Curtain).* This technology has been disclosed as using hollow polymeric membranes able to diffuse oxygen without producing bubbles going out of the water. This system, which is very efficient in terms of gas dissolution in groundwater, results to be partially limited by biofouling phenomena (biomass/bacteria adhesion), that in the course of time, determine the membrane blinding with loss of the system efficiency. In order to avoid biofouling, this technology is preferentially applied out of polluted zone, leaving the groundwater flow to transport oxygen inside the contaminated zone. This operational way induces longer remediation times and, as a consequence, an increase of costs since the velocity of the groundwater flow could be very slow.

Besides being characterised by the drawbacks described above (electric power consumption, biofouling, waste production, etc.), the systems used at present are based on sizing methods that can be improved.

The methodology used at present to define and size the *in-situ* groundwater reclamation involves the provision of several wells in the contaminated site and the analysis of groundwater samples taken from them.

Such a methodology has two important drawbacks: the first one is that the sizing method is based on data related to the analysed points, whereas the site of reclamation is a continuous system; the second one is that the analyses are set out so as to look for the presumably present substances only, since it is difficult to analyse every type of products.

In this way, only an approximate image of the pollution spread over the site can be obtained, since the concentration of contaminated substances present in the analysed wells is deducted by interpolation of the few collected data. It should be considered that usually, sampling mesh is about 20-100 m²; therefore, the effected analyses cannot be representative for the whole area.

Moreover, the analyses identify only some of the actually present pollutants and, however precise such analyses may be, they cannot take into account the ground structure and the influence of the soil on the diffusion and consumption of the gases utilised for reclamation.

These data are sometimes evaluated separately with specific tests, at different times compared with chemical analysis. It should be noted, however, that even if all the pollutants and the ground structure were evaluated, it would still be difficult, or even impossible, to determine the actual diffusion of reclamation gas in groundwater. Actually, gas diffusion into the aquifer is subject to the interference exerted by hydrogeological, chemical, physical and biological mechanisms present in the ground.

Moreover, gas diffusion has a velocity which differs from the groundwater movement and so the actual time required for obtaining the complete site decontamination is difficult to be predicted. Therefore, in order to correctly size decontamination systems based on gas injection in groundwater, both water table velocity and gas progress velocity have to be simultaneously known.

At present, these values are detected at different times and, as a consequence, project data and real data collected during reclamation often do not agree.

In order to effect a reclamation of a contaminate site, it is therefore necessary to know the site characterization. Parameters of characterisation have to describe the stratigraphy of the ground, its composition, its conducibility, the nature and concentration of pollutants, the water table velocity, its temperature, its deepness, piezometric level and so on. Such parameters are identified by the analysis of the ground and groundwater and specific hydrogeologic tests. The characterization of the site is obtained by interpolating and elaborating the data collected at the sampling point and through hydrogeologic tests.

Sometimes, pilot tests that simulate the application of the chosen technology of remediation can be carried out, so that realistic data are obtained. In the case of gas injection, the test involves the injection of gas in a well and the monitoring of the progress of contamination and hydrochemical parameters in wells/piezometer placed around such well. The sizing of the reclamation site is decided on the basis of the obtained results.

Although long times are often required for obtaining the above results, the latter are not always significant as far as the actual gas diffusion is concerned. This may be due to several factors, such as the presence of pollutants that have not been taken into account and which determine a higher gas consumption without allowing the detection of the gas in monitoring wells, the presence of substances that chemically consume the injected gas, a different hydrogeologic conformation of ground that slows down or speeds up gas velocity, etc..

Another important drawback is the water table fluctuation, which often can make the obtained results unclear. In particular, water table fluctuation can involve significant variation of pollutant concentration and so determine an over or sub-estimation of the actual gas diffusion.

As a consequence, the identification of the influence radius of injection wells, and consequently the definition of their number for real reclamation action, can be very difficult.

The difficulties met during the sizing phase can be amplified during the management of the real remediation. In fact, an apparently unexplainable fluctuation of the pollutant can be obtained, so that the real decontamination progress cannot be easily understood.

US-A-60003365 discloses a method that uses tracers to localize, identify and quantify the presence of NAPL (Non Aqueous Phase Liquids) contamination in soil and to collect data for a future remediation. It is based on use of organic or inorganic substances injected in soil and on their different partitioning in the contamination. These substances (alcohols, fluorocarbons, fluorosulfur compounds, esters, amides, hydrocarbons, sulfates, aldehydes, ketones and salts of bromide and iodide) are used as tracers. It has to be underlined that these tracers are contaminants themselves. The use of tracers according to patent US-A-60003365 requires knowledge of the pollutants.

In WO-A-2006/004567 is reported the use of tracers to measure the volume of void, filled with water, in a solid waste disposed in a landfill, using at least two tracers one reactive with water (difluoromethane) and one non reactive (helium). This method therefore is applied in a particular field (waste disposal) and with a particular goal. the tracers have the purpose to individuate the porosity (volume of void) and for this purpose it uses two tracers; the tracers are used together with the gas needed for the remediation (in WO-A-2006/004567 there is not a gas for remediation) and the gases are selected on the basis of their difference in diffusivity with the gas used in groundwater remediation.

US A 5992213 discloses a method to determine the contamination of a porous soil, trough a generation rate test based on use of gases. In particular the method suggested uses tracer gases (that in this case can be oxygen) in order to define the equilibrium achievement of the generation test. The tracers constitute an element of a sampling methodology in order to identify the level of contamination of porous soil, but is not connected to remediation technology.

### Summary of the invention

The scope of the invention consists in overcoming the above drawbacks.

Such scope is obtained through the features reported in the attached independent claim 1. Advantageous embodiments of the invention are described in the dependent claims.

The invention relates to a method for sizing and monitoring systems of gas diffusion in groundwater through the use of a gas mixture.

This method is based on the propagation difference between a gas useful for remediation and another gas and/or gases, which do not chemically or biologically react with the aquifer and with substances and the biomass present in the aquifer.

The gas mixture can be used both during the pilot test and during the actual reclamation. In the first case, important data for a correct sizing of the system can be obtained. In the second case, the reclamation progress can be controlled by identifying the presence of pollutant substances localized in the ground which were not previously taken into consideration, with a relevant estimate of concentration, or the variation of water table movement, etc..

An improved use of the gas mixtures is obtained when they are combined with laboratory tests. Such tests are useful in order to find what the consumption of remediation gas is, that is, the gas used for chemical or biological reaction with contaminants of the aquifer. The tests are carried out on one or more samples of saturated ground and/or groundwater taken from representative points of the site.

The gas mixture used for pilot test and/or remediation depends on the type of substances present in the contaminated site. The main gas constituting the mixture is represented by gas "useful" for reclamation. Such gas can be an oxidising gas or a reducing gas or, in any case, a gas reacting with substances and biomass present in the site and/or with the ground. The other gases of the mixture are represented by inert gases, which do not react either with the ground or with the groundwater and are not used biologically.

Gases used for reclamation, which are therefore potential mixture constituents, can be:
- oxidising gases: oxygen, ozone, and air;
- reducing gases: hydrogen, methane, and propane;
- gases that can react with the ground and/or can be dissolved in water and/or are in any case useful for remediation: carbon dioxide, nitrogen;
- noble gases that do not react either biologically or chemically: helium, neon, argon, krypton, xenon, and radon.

### Detailed description of the invention

The present invention relates to a method for sizing and monitoring systems of gas diffusion in groundwater through the use of gas mixture.

Such method is based on the propagation difference between a gas useful for remediation and another gas and/or gases, which do not react either chemically or biologically with the aquifer and with the substances and biomass present in the aquifer.

The gas mixture can be used either during pilot test or during real reclamation. In the former case, important data for a correct sizing of the system can be obtained. In the latter case, the reclamation progress can be controlled through the identification of the presence of localised pollutant substances which were not previously considered together with an estimate of their concentration, or the variation of water table movement, etc.

The use of the gas mixture becomes more effective if combined with laboratory tests. Such tests are useful in order to find what the consumption of remediation gas is, that is, the gas used for chemical or biological reaction with contaminants of the aquifer. Such tests are carried out on one or more samples of saturated ground and/or groundwater taken from representative points of the site.

The gas mixture used for the pilot test and/or remediation depends on the type of substances present in the contaminated site. The main gas of the mixture is represented by a gas useful for reclamation. Such gas can be an oxidising gas or a reducing gas or, in any case, a gas which reacts with the substances and the biomass present in the site and/or with the ground. The other mixture gases are represented by inert gases, which do not react either with the ground or with the groundwater and are not used biologically.

The gas mixture contains up to 99,99% vol. of a gas useful for reclamation and the remaining part consists of inert gases. Preferably, during pilot *in situ* tests, the concentration of gas useful for remediation can be up to 90%, while during reclamation it can be increased up to 95%.

Gases used for reclamation, which are therefore potential mixture constituents, can be:
- oxidising gases: oxygen, ozone, and air;
- reducing gases: hydrogen, methane, and propane;
- gases that can react with ground and/or be dissolved in water and/or be useful for remediation in someway: carbon dioxide, nitrogen;
- noble gases that do not react either biologically or chemically: helium, neon, argon, krypton, xenon, and radon.

The gas mixture used in the method according to the invention is constituted by at least one gas "useful" for remediation, for example oxygen, and, at least two tracer gases. An "inert" gas for remediation purposes is for example neon. The inert gas acts as tracer gas.

At least two tracer gases are required to better interpret data from the pilot test or the real application. When two tracer gases are present, the gas velocities in groundwater and their propagation around the injection point can be better estimated.

The diffusion depends on gas type, and therefore at least two gases as tracer gases having a diffusion velocity in water respectively lower and higher than that of the at least one "useful" gas are used.

The propagation velocity of gases in the ground obviously depends on ground porosity and water table transport velocity: while tracer gas concentrations supply direct information on velocity, the concentration of the at least one "useful" gas consumed by ground reaction supplies information on the used quantity of this gas. By comparing the obtained information with the gas concentrations measured in the monitoring wells around the injection well, and by considering the theoretical "useful" gas consumption identified during laboratory tests, it is possible to deduct: the presence of initially not considered pollutants, or, in any case, reactions that consume the at least one "useful" gas; groundwater velocity; groundwater fluctuation; time needed by the at least one "useful" gas to reach a certain area in the absence of initially not considered pollutants.

This information is essential for both the correct sizing of the system and for understanding whether further analyses of the site are required before the final remediation.

Methods used at present are not able to precisely define the above-listed parameters since, as already explained, they are based on data detected at some points on measurements of the pollutants carried out at different times with respect to the measurements of groundwater velocity, on the impossibility to continuously control groundwater movements.

It should be noted that, while the traditional methods try to size the gas injection systems using discrete measurements, the new method considers the injection effect in a "integral" and coherent way both in time and space.

The proposed method is, moreover, very useful during reclamation. The use of the gas mixture as described above provides essential information for the identification of any initially unfound localised pollutants.

Also in this case, anomalies can be checked on the same sample through the comparison of the concentrations of tracer gases and the concentration of the at least one "useful" gas.

In order to correctly apply the proposed method, the sequential steps to be carried out in a contaminated site are:
- conventional first characterisation of the site in order to define the presumed pollutants present therein, with the provision of wells for groundwater sampling;
- definition of the at least one gas "useful" for reclamation;
- execution of laboratory test on saturated ground and/or groundwater samples, representative of site contamination and hydrogeologic conditions, through treatment with the identified at least one "useful" gas;
- repetition of preferably at least three laboratory tests on saturated ground and/or groundwater samples taken from the contaminated zone;
- set up of the *in situ* pilot test through the identification of a well which is characteristic of the reclamation area and start of the injection of a gas mixture. The gas mixture has to contain the at least one "useful" gas and at least two tracer gases having a diffusivity in water similar to that of the at least one "useful" gas; the diffusivity of the at least two tracer gases should be respectively higher and lower than that of the at least one "useful" gas;
- set up of the monitoring wells around the gas mixture injection point;
- start of the injection in the selected area and sampling in the monitoring wells in order to measure the concentration of the mixture gases in groundwater and its progress in time;
- simultaneous sampling in order to analyse the pollutants concentration in groundwater and its progress in time;
- monitoring of the progress of fundamental hydrochemical parameters (pH, conducibility, temperature, redox potential, dissolved oxygen);
- monitoring of the progress of the groundwater piezometric levels;
- when all the gases present in the gas mixture reach the monitoring wells, the first evaluation can be carried out using the analysis results of gases, pollutants, hydrochemical parameters and laboratory tests. The elaboration of data allows to define: the actual consumption of the at least one "useful" gas per unit of volume and time; apparent diffusion velocity of gases; presence of pollutants different from those originally hypothesised (in terms of type and concentration) or, any other possible consumption of the at least one "useful" gas;
- determination of the influence radius of the gas injection system as a function of the specific tracer gas diffusion in the site;
- sizing of the injection system for actual reclamation, and identification of the monitoring wells for the control of the remediation progress;
- installation of the injection system as the sizing definition in the reclamation site;
- injection of the gas mixture constituted by at least one "useful" gas and at least two tracer gases in the wells;
- measurement of the mixture gases in the monitoring wells at regular intervals;
- evaluation of the progress of remediation through the comparison of the results of the analysis of gases, pollutants, laboratory tests and *in situ* pilot test; any anomalies that might prove the presence of higher or lower amounts of pollutants, and/or a hydrogeological configuration different from that originally defined can be put into evidence.

The method involves the use of gas mixtures so that, together with the gas actually used for remediation, there are at least two tracer gases which are not used either biologically or chemically.

This system allows to obtain, during the pilot test, very important data for the sizing of the process of real reclamation.

Such method, in fact, allows to understand the actual diffusion of gas in groundwater through the comparison of the progress of the tracer gases and the concentration of remediation gas used or consumed by the ground, biomass, chemical reagents, etc..

The use of a gas mixture containing at least one reacting gas and at least two tracer gases allows to ascertain whether previously unidentified pollutants are present or whether they are present in unexpected amounts, and/or whether any changes occurred as far as the hydrogeological features are concerned (for example, variation of gradient of hydraulic conducibility due to the presence of barriers and/or underground volumes of backfill).

The method of the invention can be applied in the following, non limiting cases:
- systems of air injection in groundwater through the use of compressors (air sparging/biosparging);
- systems of air injection in groundwater based on technologies of pure diffusion of gas in saturated ground (ISOC^{R}, ORC, compressed gas gurgling in groundwater);
- reclamation system for which it is desired to find the sizing and/or diffusion/dispersion model of a gas in saturated ground contaminated by organic substances;
- reclamation system for which it is desired to find the sizing and/or diffusion/dispersion model of a gas in saturated ground contaminated by inorganic substances;
- reclamation system for which it is desired to find the sizing and/or diffusion/dispersion model of a gas in saturated ground contaminated by hydrocarbon substances;
- reclamation system for which it is desired to find the sizing and/or diffusion/dispersion model of a gas in saturated ground contaminated by halogenated substances;
- reclamation system for which it is desired to find the sizing and/or diffusion/dispersion model of a gas in saturated ground contaminated by metal ions;
- reclamation system for which it is desired to find the sizing and/or diffusion/dispersion model of a gas in saturated ground contaminated by substances that are biodegraded by oxidation;
- reclamation system for which it is desired to find the sizing and/or diffusion/dispersion model of a gas in saturated ground contaminated by substances that are biodegraded by reduction;
- reclamation system for which it is desired to find the sizing and/or diffusion/dispersion model of a gas in saturated ground contaminated by chemically oxidizable substances;
- reclamation system for which it is desired to find the sizing and/or diffusion/dispersion model of a gas in saturated ground contaminated by chemically reducible substances;
- reclamation system for which it is desired to find the sizing and/or diffusion/dispersion model of a gas in saturated ground for sites with any stratrigraphic/hydrogeologic/geologic configuration;
- in general, any reclamation system for which it is desired to find the sizing and/or diffusion/dispersion model of a gas in saturated ground.

## Claims

1. A method to size and monitor the diffusion of gases present in a gas mixture
injected in groundwater to promote decontamination processes,
- the gas mixture being constituted by at least one gas useful for remediation and at least two tracer gases, wherein
- said at least one gas useful for remediation is biologically or chemically consumed to purify the groundwater from organic or inorganic contamination and said at least two tracer gases have a water diffusivity respectively higher and lower than the water diffusivity of the at least one gas useful for remediation, and are completely inert, do not react with ground and substances present therein and with the groundwater and do not introduce any pollution,
the method being based on the propagation difference between said at least one gas useful for remediation and the tracer gases, and consisting in:
- defining the influence radius of said at least one injected gas useful for the remediation;
- identifying, through elaboration of tracer gases concentration detected in monitoring wells, the presence and the quantity of pollutants in groundwater, even when such pollutants have not initially been considered.

2. The method of claim 1 applied during the reclamation, in order to control the remediation progress.

3. The method of claim 1 or 2 wherein the gas useful for remediation in the gas mixture is one of following: oxygen, ozone, air, hydrogen, methane, propane, nitrogen, and carbon dioxide.

4. The method of claims 1, 2 or 3 wherein each tracer gas in the gas mixture is one of following: helium, neon, argon, krypton, xenon, and radon.

5. The method of claim 1 applied during *in-situ* pilot tests, wherein the gas mixture composition is constituted only by tracer gases with a different water diffusivity.

6. The method of claim 1 applied during *in-situ* pilot tests, wherein the gas mixture composition is constituted at least by one gas useful for reclamation with concentration up to 99,99% and the remaining part is constituted by tracer gases.

7. The method of claim 2 applied during reclamation, in which the gas mixture composition is constituted at least by one gas useful for reclamation with concentration up to 99,99% and the remaining part is constituted by tracer gases.

8. The method of claim 1, wherein the gas diffusion is evaluated in function of the specific characteristics of the site:
- water table velocity;
- nature and concentration of pollutants;
- presence of substances that chemically consume the at least one useful gas;
- variation of hydraulic conducibility;
- presence of hydraulic barriers;

9. Method of claim 1, comprising the following steps:
- first site characterisation in order to define the presumed pollutants present through groundwater analysis taken from a certain number of wells;
- definition of the at least one gas to be used for reclamation;
- execution of laboratory tests using the defined gas and a saturated ground or groundwater, representative of the contamination and hydrogeologic conformation of the site;
- repetition of laboratory test on samples taken from reclamation area;
- identification of a characteristic well, in terms of contamination and hydrogeologic conformation of reclamation area, and injection of a gas mixture prepared according to claims 1-7 into such well;
- preparation of monitoring wells placed around the injection point;
- beginning of the injection of the gas mixture in the selected point and execution of planned samplings in monitoring wells in order to measure the concentration of the gases present in the gas mixture and the progress of the contamination;
- monitoring of fundamental hydrochemical parameters: pH, temperature, conducibility, redox potential, dissolved oxygen;
- monitoring of piezometric levels;
- evaluation of the obtained results;
- sizing of the injection system on reclamation area and definition of the monitoring wells;
- installation of an injection system in the reclamation site;
- injection of the gas mixture constituted by at least one useful gas and at least two tracer gases in the wells;
- measurement at regular intervals of mixture gases in the monitoring wells;
- evaluation of the progress of reclamation, comparing the gas and pollutant concentrations in the monitoring wells and the results of laboratory and *in-situ* tests.

## Patentansprüche

1. Verfahren zum Dimensionieren und Überwachen der Diffusion der Gasen, die in einem Gasgemisch vorliegen, welches in Grundwasser eingespritzt wird, um Dekontaminationsprozesse zu begünstigen, wobei
- das Gasgemisch aus wenigstens einem Gas, das zur Sanierung nützlich ist, und wenigstens zwei Tracer-Gasen besteht, wobei
- das wenigstens eine Gas, das zur Sanierung nützlich ist, biologisch oder chemisch verbraucht wird, um das Grundwasser von organischer oder anorganischer Kontamination zu reinigen, und die wenigstens zwei Tracer-Gase ein Wasserdiffusionsvermögen von höher bzw. niedriger als das Wasserdiffusionsvermögen des wenigstens einen Gases, das zur Sanierung nützlich ist, haben und vollständig inert sind, nicht mit Erdreich und darin vorliegenden Substanzen und dem Grundwasser reagieren und keine Verunreinigung einführen,
wobei das Verfahren auf der Ausbreitungsdifferenz zwischen dem wenigstens einem Gas, das zur Sanierung nützlich ist, und den Tracer-Gasen basiert und darin besteht, dass:
- der Einflussradius des wenigstens einen eingespritzten Gases, das für die Sanierung nützlich ist, definiert wird;
- durch Ausarbeiten der Konzentration der Tracer-Gase, die in Überwachungsbohrlöchern detektiert wird, das Vorliegen und die Menge von Verunreinigungen in Grundwasser, selbst wenn solche Verunreinigungen anfänglich nicht betrachtet wurden.

2. Verfahren gemäß Anspruch 1, das während der Wiederaufbereitung nützlich ist, um das Fortschreiten der Sanierung zu regulieren.

3. Verfahren gemäß Anspruch 1 oder 2, wobei das Gas, das zur Sanierung verwendet wird, in dem Gasgemisch eines der folgenden ist: Sauerstoff, Ozon, Luft, Wasserstoff, Methan, Propan, Stickstoff und Kohlendioxid.

4. Verfahren gemäß Anspruch 1, 2 oder 3, wobei jedes Tracer-Gas in dem Gasgemisch eines der folgenden ist: Helium, Neon, Argon, Krypton, Xenon und Radon.

5. Verfahren gemäß Anspruch 1, das während *in-situ*-Pilotversuchen angewandt wird, wobei die Gasgemisch-Zusammensetzung nur aus Tracer-Gasen mit einem unterschiedlichen Wasserdiffusionsvermögen besteht.

6. Verfahren gemäß Anspruch 1, das während *in-situ*-Pilotversuchen angewandt wird, wobei die Gasgemisch-Zusammensetzung wenigstens von einem Gas, das zur Wiederaufbereitung nützlich ist, mit einer Konzentration von bis zu 99,99% gebildet wird und der restliche Teil durch Tracer-Gase gebildet wird.

7. Verfahren gemäß Anspruch 2, das während einer Wiederaufbereitung angewandt wird, in dem die Gasgemisch-Zusammensetzung wenigstens durch ein Gas, zur Wiederaufbereitung nützlich ist, mit einer Konzentration von bis zu 99,99% gebildet wird und der restliche Teil durch Tracer-Gase gebildet wird.

8. Verfahren gemäß Anspruch 1, wobei die Gasdiffusion als Funktion der spezifischen Merkmale der Stelle evaluiert wird:
- Grundwasserspiegel-Geschwindigkeit;
- Natur und Konzentration von Verunreinigungen;
- Vorliegen von Substanzen, die das wenigstens eine nützliche Gas chemisch verbrauchen;
- Schwankung der hydraulischen Leitfähigkeit;
- Vorliegen von hydraulischen Barrieren.

9. Verfahren gemäß Anspruch 1, umfassend die folgenden Schritte:
- Erststellen-Charakterisierung, um die vermuteten vorliegenden Verunreinigungen durch Analyse von Grundwasser, das aus einer bestimmten Anzahl von Borlöchern entnommen wurde, zu definieren;
- Definition des wenigstens einen Gases, das zur Wiederaufbereitung zu verwenden ist;
- Ausführung von Laborversuchen unter Verwendung des definierten Gases und eines gesättigten Erdreichs oder Grundwassers, repräsentativ für die Kontamination und hydrogeologische Konformation der Stelle;
- Wiederholung von Laborversuch an Proben, die aus einem Wiederaufbereitungsbereich entnommen wurden;
- Identifizierung eines charakteristischen Bohrlochs als Kontamination und hydrogeologische Konformation des Wiederaufbereitungsbereichs und Einspritzung eines Gasgemisches, das gemäß Anspruch 1 bis 7 hergestellt worden war, in ein solches Bohrloch;
- Herstellung von Überwachungsbohrlöchern, die um den Einspritzungspunkt angelegt wurden;
- Beginnen mit der Einspritzung des Gasgemisches in den ausgewählten Punkt und Ausführung geplanter Probenentnahme in Überwachungsbohrlöchern, um die Konzentration der Gase, die in dem Gasgemisch vorliegt, und das Fortschreiten der Kontamination zu messen;
- Überwachen fundamentaler hydrochemischer Parameter: pH, Temperatur, Leitfähigkeit, Redoxpotential, gelöster Sauerstoff;
- Überwachen der piezometrischen Level;
- Evaluierung der erhaltenen Resultate;
- Dimensionieren des Einspritzsystems in Wiederaufbereitungsbereich und Definition der Überwachungsbohrlöcher;
- Einbau eines Einspritzsystems an der Wiederaufbereitungsstelle;
- Einspritzung des Gasgemisches, das aus wenigstens einem nützlichen Gas und wenigstens zwei Tracer-Gasen besteht, in die Bohrlöcher;
- Messung von Gasgemischen in den Überwachungsbohrlöchern in regelmäßigen Intervallen;
- Evaluierung des Fortschritts der Wiederaufbereitung, wobei die Gas- und Verunreinigungskonzentrationen in den Überwachungsbohrlöchern und die Resultate von Labor- und *in-situ-*Versuchen verglichen werden.

## Revendications

1. Procédé de dimensionnement et de surveillance de la diffusion des gaz présents dans un mélange gazeux injecté dans les eaux souterraines pour favoriser des processus de décontamination,
- le mélange gazeux étant constitué d'au moins un gaz utile à la remédiation et d'au moins deux gaz de traçage, dans lequel
- ledit au moins un gaz utile à la remédiation est biologiquement ou chimiquement consommé pour purifier les eaux souterraines de la contamination organique ou inorganique et lesdits au moins deux gaz de traçage présentent une diffusivité hydrique respectivement supérieure et inférieure à celle de l'au moins un gaz utile à la remédiation, et sont complètement inertes, ne réagissent pas avec le sol et les substances qui y sont présentes et avec les eaux souterraines et n'introduisent pas de pollution,
le procédé étant fondé sur la différence de propagation entre ledit au moins un gaz utile à la remédiation et les gaz de traçage, et consistant à :
- définir le rayon d'influence dudit au moins un gaz injecté utile à la remédiation ;
- identifier, par élaboration de la concentration des gaz de traçage détectée dans les puits de surveillance, la présence et la quantité de polluants dans les eaux souterraines, même lorsque de tels polluants n'ont pas été pris en considération au départ.

2. Procédé selon la revendication 1 appliqué pendant la récupération, afin de contrôler la progression de la remédiation.

3. Procédé selon la revendication 1 ou 2 dans lequel le gaz utile à la remédiation dans le mélange gazeux est l'un des suivants : l'oxygène, l'ozone, l'air, l'hydrogène, le méthane, le propane, l'azote, et le dioxyde de carbone.

4. Procédé selon les revendications 1, 2 ou 3 dans lequel chaque gaz de traçage dans le mélange gazeux est l'un des suivants : l'hélium, le néon, l'argon, le krypton, le xénon, et le radon.

5. Procédé selon la revendication 1 appliqué pendant des essais pilotes *in situ,* dans lequel la composition du mélange gazeux n'est constituée que par des gaz de traçage présentant une diffusivité hydrique différente.

6. Procédé selon la revendication 1 appliqué pendant des essais pilotes *in situ,* dans lequel la composition du mélange gazeux est constituée au moins par un gaz utile à la récupération avec une concentration jusqu'à 99,99 % et la partie restante est constituée par des gaz de traçage.

7. Procédé selon la revendication 2 appliqué pendant la récupération, dans lequel la composition du mélange gazeux est constituée au moins par un gaz utile à la récupération avec une concentration jusqu'à 99,99 % et la partie restante est constituée par des gaz de traçage.

8. Procédé selon la revendication 1, dans lequel la diffusion de gaz est évaluée en fonction des caractéristiques spécifiques du site :
- vitesse de la nappe phréatique ;
- nature et concentration des polluants ;
- présence de substances qui consomment chimiquement l'au moins un gaz utile ;
- variation de la conductibilité hydraulique ;
- présence de barrières hydrauliques ;

9. Procédé selon la revendication 1, comprenant les étapes suivantes :
- première caractérisation du site afin de définir les polluants présumés présents par une analyse des eaux souterraines provenant d'un certain nombre de puits ;
- définition de l'au moins un gaz à utiliser pour la récupération ;
- réalisation d'essais en laboratoire en utilisant le gaz défini et un sol ou des eaux souterraines saturés, représentatifs de la contamination et de la conformation hydrogéologique du site ;
- répétition des essais en laboratoire sur des échantillons prélevés dans la zone de récupération ;
- Identification d'un puits caractéristique, du point de vue de la contamination et de de la conformation hydrogéologique de la zone de récupération, et injection d'un mélange gazeux préparé conformément aux revendications 1-7 dans un tel puits ;
- préparation de puits de surveillance placés autour du point d'injection ;
- début de l'injection du mélange gazeux dans le point choisi et réalisation d'échantillonnages planifiés dans les puits de surveillance afin de mesurer la concentration des gaz présents dans le mélange gazeux et la progression de la contamination ;
- surveillance des paramètres hydrochimiques fondamentaux : pH, température, conductibilité, potentiel redox, oxygène dissous ;
- surveillance des niveaux piézométriques ;
- évaluation des résultats obtenus ;
- dimensionnement du système d'injection sur la zone de récupération et définition des puits de surveillance ;
- installation d'un système d'injection dans le site de récupération ;
- injection du mélange gazeux constitué par au moins un gaz utile et au moins deux gaz de traçage dans les puits ;
- mesurage à intervalles réguliers des gaz du mélange dans les puits de surveillance ;
- évaluation de la progression de la récupération, en comparant les concentrations de gaz et de polluants dans les puits de surveillance et les résultats des essais en laboratoire et *in situ.*
